# EUROPEAN PATENT SPECIFICATION

(11) **EP 2 050 444 B1**
(45) Date of publication and mention of the grant of the patent: **11.05.2011**
(21) Application number: 07744852.0
(22) Date of filing: 07.06.2007
(51) Int. Cl.: A61K 31/198, A23L 1/305, A61K 38/00, A61P 3/02

(54) **FATIGUE-REDUCING AGENT**
MITTEL ZUR VERRINGERUNG VON MÜDIGKEIT
AGENT DE RÉDUCTION DE LA FATIGUE

(30) Priority: 07.06.2006 JP 2006158156
(43) Date of publication of application: 22.04.2009
(73) Proprietor: Kyowa Hakko Bio Co., Ltd., Tokyo, 100-8185 (JP)
(72) Inventor: KOMATSU, Miho, Tsukuba-shi, Ibaraki 305-0841 (JP); MORISHITA, Koji, Tsukuba-shi, Ibaraki 305-0841 (JP); SHIBASAKI, Takeshi, Tsukuba-shi, Ibaraki 305-0841 (JP); TODA, Itaru, Tsukuba-shi, Ibaraki 305-0841 (JP)
(74) Representative: Dossmann, Gérard
(86) International application number: PCT/JP2007/061522
(87) International publication number: WO 2007/142286

(56) References cited:
- EP-A- 1 935 418
- WO-A-2004/071435
- WO-A1-2004/078171
- AU-A- 3 460 571
- JP-A- 08 275 752
- JP-A- 2002 338 473
- JP-A- 2004 526 793
- JP-B1- 42 007 767
- US-A1- 2004 001 817
- NOVELLI G P ET AL: "Exogenous glutathione increases endurance to muscle effort in mice" PHARMACOLOGICAL RESEARCH, ACADEMIC PRESS, LONDON, GB, vol. 23, no. 2, 1 January 1991 (1991-01-01), pages 149-155, XP003007663 ISSN: 1043-6618
- NOVELLI G.P. ET AL.: 'Exogenous glutathione increases endurance to muscle effort in mice' PHARMACOL. RES. vol. 23, no. 2, 1991, pages 149 - 155, XP003007663

## Description

### Technical Field

The present invention relates to an agent comprising ornithine or a salt thereof and glutathione or a salt thereof as active ingredients for use as a fatigue-reducing agent

### Background Art

Ornithine is used as a food material which promotes secretion of growth hormone to enhance muscle synthesis or which enhances basal metabolism to prevent obesity mainly in the US and Japan. Ornithine is also used as a drug which improves liver disorders in the form of L-ornithine L-aspartate in Europe.

It is known that sickly fatigue associated with the rise of the ammonia level in blood is improved by administering ornithine or a salt thereof to lower the ammonia level in blood (patent document Nos. 1 to 3 and non-patent document No. 1). It is also known that subjective symptoms of fatigue of a healthy person are alleviated by ornithine ingestion (non-patent document No. 2).

However, the fatigue-reducing activity of glutathione is not known, and it is not known either that a combination of ornithine or a salt thereof and glutathione or a salt thereof has a synergistic effect to reduce fatigue.
Patent document No. 1:
   Japanese Published Examined Patent Application No. 7767/67
Patent document No. 2:
   Japanese Published Examined Patent Application No. 3194/71
Patent document No. 3:
   Japanese Published Examined Patent Application No. 8592/66
Non-patent document No. 1:
   Arzneim.-Forsch. (Drug Res.), Vol. 8, p. 1064-1067 (1970)
Non-patent document No. 2:
   Shokuhin to Kaihatsu (Foods and Development), Vol. 40, No. 11, p. 62-64 (2005)

### Disclosure of the Invention

### Problems to be Solved by the Invention

There exists a demand for drugs, functional foods or the like which reduce fatigue of a tired person and enable him to lead a fulfilled life. That is, an object of the present invention is to provide a fatigue-reducing agent.

### Means for Solving the Problems

The present invention relates to the following (1) to (3).
(1) an agent comprising, as active ingredients, ornithine or a salt thereof and glutathione or a salt thereof for use as a fatigue-reducing agent
(2) Use of ornithine or a salt thereof and glutathione or a salt thereof for reducing fatigue
(3) Use of ornithine or a salt thereof and glutathione or a salt thereof for the manufacture of a fatigue-reducing agent.

### Effect of the Invention

The present invention provides a safe and effective agent comprising, as active ingredients, ornithine or a salt thereof and glutathione or a salt thereof for use as a fatigue-reducing agent

### Brief Description of the Drawings

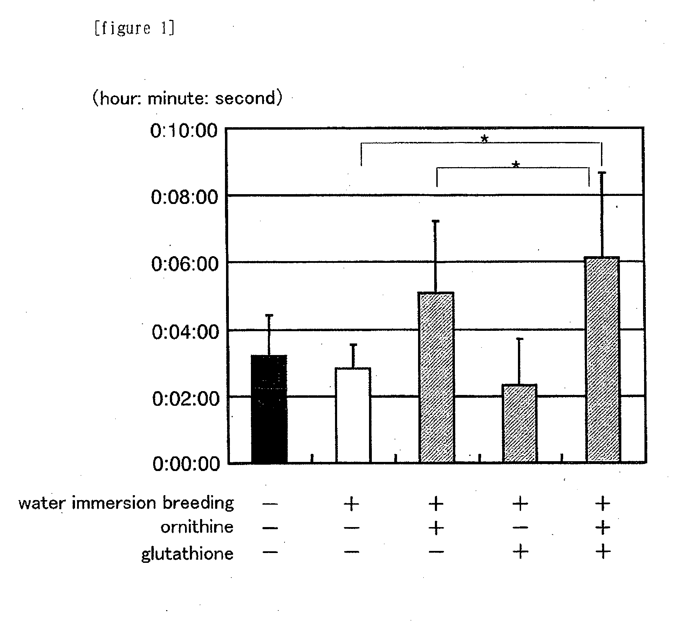

[Fig. 1] Fig. 1 is a graph showing the swimming time of rats bred under different conditions. "+" and "-" indicate whether or not a breeding condition shown in the leftmost column is applied. "*" indicates that the difference is significant at the level of less than 5%.

### Best Modes for Carrying Out the Invention

Ornithine used in the present invention includes L-ornithine and D-ornithine, and preferred is L-ornithine.

Ornithine used in the present invention may be obtained by any process. L-Ornithine can be obtained, for example, by the chemical synthesis method [Coll. Czechoslov. Chem. Commun., 24, 1993 (1959)] and the fermentation method (Japanese Published Unexamined Patent Application No. 24096/78 and Japanese Published Unexamined Patent Application No. 119194/86).

L-Ornithine and D-ornithine can also be purchased from Sigma-Aldrich Corporation.

Examples of the salts of ornithine include acid addition salts, metal salts, ammonium salts, organic amine addition salts and amino acid addition salts.

Examples of the acid addition salts include inorganic acid addition salts such as hydrochloride, sulfate, nitrate and phosphate, and organic acid addition salts such as acetate, maleate, fumarate, citrate, malate, lactate, α-ketoglutarate, gluconate and caprylate.

Examples of the metal salts are alkali metal salts such as sodium salt and potassium salt, alkaline earth metal salts such as magnesium salt and calcium salt, aluminum salt and zinc salt.

Examples of the ammonium salts are ammonium salt and tetramethylammonium salt.

Examples of the organic amine addition salts are salts with morpholine and piperidine.

Examples of the amino acid addition salts are salts with glycine, phenylalanine, lysine, aspartic acid and glutamic acid.

Among the above salts of ornithine, hydrochloride, citrate, malate, α-ketoglutarate and aspartate are preferably used, but the other salts or appropriate combinations of more than two salts may be used.

Glutathione used in the present invention includes reduced glutathione and oxidized glutathione.

Reduced glutathione refers to a tripeptide having the γ-L-Glu-L-Cys-Gly structure, and oxidized glutathione refers to a glutathione dipeptide in which two molecules of reduced glutathione are linked by an SS bond.

The reduced glutathione and the oxidized glutathione used in the present invention may be obtained by any production process. Examples of the processes for the production of reduced glutathione include the extraction method from microorganisms such as yeast [Methods in Enzymology, 3, 603 (1957)], the chemical synthesis method [Bull. Chem., Soc. Jpn., 53, 2529 (1980)] and the enzymatic method (Japanese Published Unexamined Patent Application No. 74595/86), and an example of the process for the production of oxidized glutathione is the method of Acta Biochim. Pol., 17, 175 (1970). Reduced glutathione and oxidized glutathione can also be purchased from Sigma-Aldrich Corporation.

The agent of the present invention for use as a fatigue-reducing agent may comprise either reduced glutathione or oxidized glutathione alone or may simultaneously comprise reduced glutathione and oxidized glutathione.

The reduced glutathione and the oxidized glutathione contained in the fatigue-reducing agent of the present invention may exist as salts thereof in the agent. Examples of the salts of reduced glutathione and oxidized glutathione include the same salts as those of ornithine as mentioned above.

The composition ratio of ornithine or a salt thereof to glutathione or a salt thereof in the fatigue-reducing agent of the present invention is 1:100 to 100:1, preferably 1:50 to 50:1, particularly preferably 10:1 to 1:10 by weight.

The agent of the present invention for use as a fatigue-reducing agent may be prepared in such a way that ornithine or a salt thereof and glutathione or a salt thereof are contained in the same preparation, or they are contained in separate preparations, which are used as a kit or a set (hereinafter referred to merely as a kit).

The preparations contained in a kit, etc. may be in any form so long as they exist separately. For example, they may be wrapped separately or contained in the same container.

When the preparations contained in a kit are administered or ingested separately, it is desirable that they are administered within the time period in which the active ingredients in the preparations are highly effective in the body. For example, at one administration or intake, all the preparations are administered or ingested within 8 hours, preferably within 2 hours.

Fatigue can be reduced by administering or ingesting the fatigue-reducing agent of the present invention.

Fatigue includes physiological fatigue and sickly fatigue.

Physiological fatigue refers to defense responses to maintain human health. More specifically, it refers to fatigue caused by ordinary activities such as works, household works and leisure sports, and includes not only physical fatigue but also mental fatigue.

On the other hand, slickly fatigue refers to that caused as symptoms accompanying pre-existing diseases such as heart disease, bronchial asthma, hepatitis, anemia, metabolic disease, muscle disease, various infectious diseases and cancers, and also fatigue in chronic fatigue syndrome, depression induced by mental factors, over training syndrome caused by sports, etc.

The agent of the present invention for use as a fatigue-reducing agent can be used for reducing any of the above fatigue.

In the agent of the present invention for use as a fatigue-reducing agent ornithine or a salt thereof and glutathione or a salt thereof can be administered as such, but it is usually preferred to provide the agent as various kinds of preparations.

The preparations comprise, as active ingredients, ornithine or a salt thereof and glutathione or a salt thereof, and may further comprise arbitrary active ingredients. The preparations are produced according to arbitrary methods well known in the technical field of pharmaceutics by mixing the active ingredients with one or more kinds of pharmaceutically acceptable carriers.

It is desirable to administer the preparation by the route that is the most effective for the reduction of fatigue. Suitable administration routes include oral administration and non-oral administration such as intravenous administration, intraperitoneal administration or subcutaneous administration. Preferred is oral administration.

The preparation may be administered either in the form of oral preparations (e.g., tablets, powders, granules, pills, suspensions, emulsions, infusions/decoctions, capsules, syrups, liquids, elixirs, extracts, tinctures and fluid extracts) or non-oral preparations (e.g., injections, drops, creams and suppositories). Preferred are oral preparations.

Liquid preparations suitable for oral administration such as syrups can be prepared by adding water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), preservatives (e.g., paraoxybenzoic acid derivatives such as methyl paraoxybenzoate, and sodium benzoate), flavors (e.g., strawberry flavor and peppermint), and the like.

Tablets, powders, granules, etc. suitable for oral administration can be prepared by adding excipients such as sugars (e.g., lactose, white sugar, glucose, sucrose, mannitol and sorbitol), starch (e.g., potato starch, wheat starch and corn starch), inorganic substances (e.g., calcium carbonate, calcium sulfate, sodium hydrogencarbonate and sodium chloride), crystalline cellulose and plant powders (e.g., licorice powder and gentian powder), disintegrating agents such as starch, agar, gelatin powder, crystalline cellulose, carmellose sodium, carmellose calcium, calcium carbonate, sodium hydrogencarbonate and sodium alginate, lubricants such as magnesium stearate, talc, hydrogenated vegetable oil, macrogol and silicone oil, binders such as polyvinyl alcohol, hydroxypropyl cellulose, methyl cellulose, ethyl cellulose, carmellose, gelatin and starch paste, surfactants such as fatty acid esters, plasticizers such as glycerin, and the like.

The preparations suitable for oral administration may also comprise additives generally used in foods and drinks such as sweeteners, coloring agents, preservatives, thickening stabilizers, antioxidants, color developers, bleaching agents, fungicides, gum bases, bitter agents, enzymes, glazing agents, sour agents, seasonings, emulsifiers, nutrient supplements, manufacture facilitating agents, flavors and spice extracts.

The preparations suitable for oral administration may be used as foods and drinks such as a health food, a functional food, a food supplement and a food for specified health uses for reducing fatigue, as such or in any of the forms such as a powder food, a sheet-shaped food, a bottled food, a canned food, a retort pouched food, a capsule food, a tablet food, a liquid food and a health drink.

Preparations suitable for non-oral administration such as injections preferably comprise a sterilized aqueous preparation containing ornithine or a salt thereof and glutathione or a salt thereof which is isotonic to the recipient's blood. In the case of an injection, for example, a solution for injection is prepared using a carrier comprising a salt solution, a glucose solution, or a mixture of a salt solution and a glucose solution.

One or more kinds of auxiliary components selected from the above-described antiseptics, preservatives, excipients, disintegrating agents, lubricants, binders, surfactants, plasticizers, etc. for oral preparations can also be employed in these non-oral preparations.

The concentration of ornithine or a salt thereof and glutathione or a salt thereof in the agent of the present invention for use as fatigue-reducing agent is appropriately determined according to the kind of the preparation, the effect expected by the administration of the preparation, etc. Ornithine or a salt thereof and glutathione or a salt thereof are usually contained in an amount of 0.1 to 100% by weight, preferably 0.5 to 70% by weight, particularly preferably 1 to 50% by weight.

When the agent of the present invention for use as a fatigue-reducing agent is administered to a human, the dose and administration schedule vary depending upon the administration route, the subject's age and body weight, etc. Usually, the agent is administered once to several times per day in an amount to give a daily dose of 50 mg to 30 g, preferably 100 mg to 10 g, particularly preferably 200 mg to 3 g per adult in terms of ornithine or a salt thereof and glutathione or a salt thereof.

There is no specific restriction as to the period of administration, but it is usually one day to one year, preferably one week to three months.

The agent according to the present invention can be used not only for humans but also for animals other than humans (hereinafter abbreviated as nonhuman animals).

Nonhuman animals include animals other than humans such as mammals, birds, reptiles, amphibians and fish.

In the case of administration to a nonhuman animal, the dose varies depending upon the age and kind of the animal, and the nature or degree of severeness of the symptom. Usually, the agent is administered once to several times per day in an amount to give a daily dose of 1 mg to 600 mg, preferably 2 mg to 200 mg, more preferably 4 mg to 60 mg per kg of body weight in terms of ornithine or a salt thereof and glutathione or a salt thereof.

There is no specific restriction as to the period of administration, but it is usually one day to one year, preferably one week to three months.

The fatigue-reducing effect of ornithine and/or glutathione is shown in the following test example.

### Test Example

SD male rats (seven-week-old; purchased from Japan SLC, Inc.) were divided into 5 groups. After 2 days of acclimation, one group was bred on an ordinary floor, and the other 4 groups were bred under water immersion for 5 days. "Water immersion breeding" refers to breeding in a plastic cage containing water at a depth of 1.5 cm. Under this environment, rats which dislike water can not get enough sleep or take a resting position and they are in a continuous restless situation mentally and physically. It is known that there appears an individual that dies when water immersion breeding is carried out for 7 days or more. A rat which underwent 5 days of water immersion breeding is said to be an animal model of exhausting fatigue leading to overwork death.

From the first day of water immersion breeding, distilled water, ornithine (L-ornithine hydrochloride; Kyowa Hakko Kogyo Co., Ltd., dose: 1000 mg/kg body weight), reduced glutathione [glutathione (reduced type); Kyowa Hakko Kogyo Co., Ltd., dose: 800 mg/kg body weight], or ornithine (dose: 500 mg/kg body weight) and reduced glutathione (dose: 400 mg/kg body weight) were orally administered once a day in an amount of 10 ml/kg body weight.

After 5 days of water immersion breeding, the rats were forced to swim with a burden of ca. 8% of their body weight, and the time until the tip of nose was submerged in water for 10 seconds or more was measured to evaluate the degree of fatigue.

The values were shown in terms of average value ± standard deviation (n=4) and a test of statistical significance was performed by two-sample t-test (two-sided distribution).

The results are shown in Figure 1. The swimming time of the rats bred under water immersion was shortened by ca. 20% compared with that of the rats bred on the floor. The recovery of the swimming time was recognized in the ornithine-administered group, but no change was recognized in the glutathione-administered group. Further, the swimming time of the group administered with a combination of ornithine and glutathione was significantly extended compared with that of the ornithine-administered group, in spite that the administered amount of ornithine is a half of that of the ornithine-administered group.

From the above results, it was revealed that a synergistic effect to reduce fatigue is obtained by combining ornithine and glutathione.

Certain embodiments of the present invention are illustrated in the following examples.

### Example 1

### Production of Tablets Comprising Ornithine and Glutathione

Ornithine hydrochloride (76.2 kg), reduced glutathione (60.0 kg), microcrystalline cellulose (36.0 kg), sucrose fatty acid ester (6.6 kg), calcium phosphate (1.2 kg) and β-cyclodextrin (20.0 kg) are mixed. The obtained mixture is compressed and molded using a rotary compression molding machine to produce tablets (diameter: 8 mm, 250 mg).

### Example 2

### Production of Enteric Capsules Comprising Ornithine and Glutathione

The mixture prepared in Example 1 (20 kg) and silicon dioxide (0.2 kg) are mixed with stirring, and the obtained mixture is put in a capsule filler and packed in No. 2 hard gelatin capsules (20000 capsules) to obtain hard capsules. The surfaces of the obtained capsules are coated with a zein solution to produce enteric capsules (20000 capsules).

### Example 3

### Production of Enteric Tablets Comprising Ornithine and Glutathione

The surfaces of the tablets prepared in Example 1 are coated with a shellac solution to produce enteric tablets.

### Example 4

### Production of Drink Comprising Ornithine and Glutathione

Ornithine hydrochloride (0.64 kg), oxidized glutathione (0.6 kg), erythritol (3 kg), citric acid (0.05 kg), artificial sweetener (3 g) and flavor (0.06 kg) are dissolved with stirring in water (50 L) at a liquid temperature of 70ºC. After being adjusted to pH 3.3 with citric acid, the solution is sterilized by a plate sterilizer and packed in bottles, followed by sterilization using a pasteurizer to produce drinks.

### Industrial Applicability

The present invention provides a safe and effective fatigue-reducing agent comprising, as active ingredients, ornithine or a salt thereof and glutathione or a salt thereof.

## Claims

1. An agent comprising, as active ingredient, ornithine or a salt thereof and glutathione or a salt thereof for use as a fatigue-reducing agent.

2. Agent comprising ornithine or a salt thereof and glutathione or a salt thereof contained in the same preparation or in separate preparations for its administration, optionally separately within eight hours, to a subject in need thereof for use as a fatigue-reducing agent.

3. Use of ornithine or a salt thereof and glutathione or a salt thereof for the manufacture of a fatigue-reducing agent.

## Patentansprüche

1. Mittel, das als Wirkstoff Ornithin oder ein Salz davon sowie Glutathion oder ein Salz davon umfasst, zur Verwendung als Mittel zur Verringerung von Müdigkeit.

2. Mittel, das Ornithin oder ein Salz davon sowie Glutathion oder ein Salz davon in demselben Präparat oder in getrennten Präparaten umfasst, zu seiner Verabreichung, optional getrennt voneinander innerhalb von acht Stunden, an einen Patienten, der dieses zur Verwendung als Mittel zur Verringerung von Müdigkeit braucht.

3. Verwendung von Ornithin oder einem Salz davon sowie Glutathion oder einem Salz davon zur Herstellung eines Mittels zur Verringerung von Müdigkeit.

## Revendications

1. Agent comprenant, comme ingrédients actifs, de l'ornithine ou de l'un de ses sels et du glutathion ou de l'un de ses sels, pour utilisation en tant qu'agent de réduction de la fatigue.

2. Agent comprenant de l'ornithine ou de l'un de ses sels et du glutathion ou de l'un de ses sels, contenus dans la même préparation ou dans des préparations distinctes en vue de leur administration à un sujet qui en a besoin, laquelle administration peut, en option, être effectuée séparément à 8 heures d'intervalle au plus, pour utilisation en tant qu'agent de réduction de la fatigue.

3. Utilisation de l'ornithine ou de l'un de ses sels et du glutathion ou de l'un de ses sels, en vue de la fabrication d'un agent de réduction de la fatigue.
